# FASCICULE DE BREVET EUROPEEN

(11) **EP 0 748 214 B1**
(45) Date de publication et mention de la délivrance du brevet: **07.10.1998**
(21) Numéro de dépôt: 95910590.9
(22) Date de dépôt: 23.02.1995
(51) Int. Cl.: A61K 9/127

(54) **LIPOSOMES, ET LEUR UTILISATION NOTAMMENT POUR L'OBTENTION DE COMPOSITIONS IMMUNOMODULATRICES**
LIPOSOMEN UND DEREN VERWENDUNG INSBESONDERE ZUR HERSTELLUNG IMMUNOMODULATORISCHER MITTEL
LIPOSOMES AND USE THEREOF ESPECIALLY TO OBTAIN IMMUNOMODULATING COMPOSITIONS

(30) Priorité: 23.02.1994 FR 9402070
(43) Date de publication de la demande: 18.12.1996
(73) Titulaire: INSTITUT PASTEUR, 75724 Paris Cédex 15 (FR); INSTITUT PASTEUR DE LILLE, 59019 Lille Cédex (FR); INSTITUT NATIONAL DE LA SANTE ET DE LA RECHERCHE MEDICALE (INSERM), 75654 Paris Cédex 13 (FR)
(72) Inventeur: RIVEAU, Gilles, F-59133 Phalempin (FR); CAPRON, André, F-59133 Phalempin (FR); IVANOFF, Nathalie, F-59000 Lille (FR); GRZYCH, Jean-Marie, F-59162 Ostricourt (FR); PHILLIPS, Nigel, Pointe Claire, Québec H9R 2GA (CA)
(74) Mandataire: Vialle-Presles, Marie José
(86) Numéro de dépôt international: FR9500215
(87) Numéro de publication internationale: WO9522961

(56) Documents cités:
- WO-A-90/04412
- WO-A-92/20370
- WO-A-93/10763
- WO-A-93/23016

## Description

La présente Invention est relative à des liposomes utilisables comme véhicules de principes actifs et immunoadjuvants pour l'obtention de compositions immunomodulatrices, en particulier de compositions vaccinales.

Les liposomes sont des vésicules dans lesquelles une phase lipidique constituée par une bicouche de molécules amphiphiles, telles que des phospholipides ou du cholestérol, emprisonne une phase interne aqueuse. Les liposomes unilamellaires comprennent une seule bicouche lipidique ; les liposomes multilamellaires comprennent plusieurs bicouches lipidiques concentriques, et la phase aqueuse constitue dans ce cas, le noyau central du liposome et la séparation entre les bicouches lipidiques.

L'utilisation des liposomes dans l'industrie pharmaceutique, comme vecteur de diverses molécules d'intérêt biologique, a fait l'objet de nombreux travaux.

En particulier, il a été proposé d'utiliser des liposomes à la fois comme vecteur d'antigène et comme immunoadjuvant pour l'obtention de vaccins. En effet alors que les liposomes ne sont pas immunogéniques en eux même, il a été constaté qu'ils augmentaient, lorsqu'ils étaient charges avec des antigènes, la réponse immune par rapport à celle observée avec l'antigène libre, voire qu'ils induisaient une réponse immune contre un antigène non immunogène en lui-même. Cet effet immunoadjuvant a été observé indépendamment de la composition des liposomes, de l'espèce animale sur laquelle ont été effectués les essais d'immunisation, et de la voie d'administration. D'après les travaux cités dans une revue bibliographique sur ce sujet, [GREGORIADIS, Immunology Today, 11, p 89-97 (1990)], cet effet apparaît comme essentiellement quantitatif, le profil isotypique des anticorps sériques induits par l'injection d'un antigène encapsulé dans des liposomes demeurant le même que celui des anticorps sériques induits par l'injection du même antigène libre.

Une revue de PHILLIPS [Bull. Inst. Pasteur, 90, 205-230, (1992)] cite différents travaux concernant l'effet immunoadjuvant de liposomes, comparé à celui de l'alun et de muramyl dipeptides, dans des préparations antigéniques administrées par injection chez la souris ; l'antigène utilisé est soit la Serum Albumine Bovine, soit un peptide. Dans ces conditions d'administration, les liposomes stimulent préférentiellement la réponse IgG, et en modifient le profil isotypique, en augmentant la proportion des IgG2a, IgG2b et IgG3 par rapport aux IgG1.

La Demande Internationale PCT WO 90/04412 décrit des préparations à base d'interleukine-2 (IL-2), encapsulée dans des liposomes, formés par exemple de DMPC et DMPG. Ces préparations peuvent être utilisées comme immunoadjuvants, en association avec divers antigènes.

Cependant, les propriétés immunoadjuvantes de ces préparations sont attribuées essentiellement à l'IL-2 ; les liposomes utilisés jouent simplement le rôle de vecteurs, permettant d'augmenter l'activité et la biodisponibilité de l'IL-2, et d'en diminuer la toxicité, mais il n'est ni indiqué ni suggéré qu'ils puissent possèder en eux-mêmes des propriétés immunoadjuvantes.

D'autres auteurs ont décrit l'effet de préparations antigéniques sous forme de liposomes, administrées par voie muqueuse, sur la réponse sécrétoire :
- MICHALEK et al. [Vaccine Research, 1:3, pp. 241-247 (1992)] ont décrit l'incorporation de différents antigènes de *S*. *mutans* dans des liposomes DPPC/cholestérol/dicétylphosphate, et ont observé, après administration orale de ces compositions, une nette augmentation de la réponse IgA sécrétoire contre ces antigènes ;
- ABRAHAM et al. [Vaccine 10:7, pp. 461-467, (1992)] ont incorporé des antigènes polysaccharidiques de *P*. *aeruginosa*, *A*. *levanicum*, et *S*. *pneunomiae* dans des liposomes cholestérol/PC/PS, et ont administré par voie intranasale les compositions obtenues. Ils ont également observé une augmentation de la réponse sécrétoire dirigée contre ces antigènes. En revanche, aucune influence sur la réponse anticorps systémique n'a été observée dans ces conditions.

Un problème qui se pose lors de l'utilisation *in vivo* de liposomes est celui de la stabilité de ceux-ci, afin que la structure liposomale soit maintenue jusqu'aux cellules cibles.

Dans le cas d'une immunisation par injection, les lipoprotéines à haute densité (HDL) du plasma, captent les phospholipides, ce qui provoque la désintégration de la structure liposomale, et ce d'autant plus rapidement que la bicouche phospholipidique est plus fluide. Lors de l'administration par voie orale, ce sont les enzymes digestives, et en particulier les phospholipases qui provoquent la dégradation des liposomes. On cherche habituellement à éviter cette dégradation en utilisant des liposomes comprenant entre 30 et 50% de cholestérol, qui résiste à l'attaque des phospholipases, et confère également une plus grande rigidité à la bicouche lipidique.

Toutefois, le cholestérol présente l'inconvénient d'être obtenu par extraction à partir de produits naturels, ce qui augmente les risques de contamination, et possède en outre certaines propriétés immunosuppressives.

Les Inventeurs ont maintenant mis au point des formulations de liposomes qui, lorsqu'ils sont utilisés pour l'encapsulation d'antigènes, peuvent induire une réponse immunologique non seulement quantitativement mais également qualitativement différente de celle induite par l'antigène libre.

Lorsque des compositions vaccinales formulées à partir des liposomes conformes à l'Invention sont administrées par voie muqueuse, elles induisent non seulement une réponse sécrétoire, mais également une réponse systémique, à la différence des compositions de l'art antérieur (par exemple celles décrites par ABRAHAM et al.). En outre, les liposomes conformes à l'Invention permettent d'augmenter tout particulièrement la réponse de tue Th2, ce qui se traduit chez la souris (et ce, indépendamment de la souche de souris qui a fait l'objet de l'immunisation), par une modification du profil isotypique de la réponse sérique en faveur des IgG1 et des IgG2b, et par une augmentation de la réponse IgA sécrétoire.

La présente Invention a pour objet l'utilisation d'une préparation de liposomes comprenant au moins une bicouche lipidique, constituée par un mélange d'un phospholipide (I) dont la température de fluidité est comprise entre 20 et 25°C, et d'un phospholipide (II) dont la température de fluidité est comprise entre 20° et 60°C, le rapport molaire entre le phospholipide (I) et le phospholipide (II) étant compris entre 5:95 et 30:70 en tant que principe actif immunoadjuvant pour l'obtention de vaccins.

Par "température de fluidité" d'un phospholipide, on entend la température à laquelle s'effectue la transition de l'état gélifié (ou solide) à l'état de cristaux liquides (état fluide) d'une bicouche dudit phospholipide.

Selon un mode de mise en oeuvre préféré de la présente Invention, le phospholipide (I) est le dimyristoylphosphatidylglycérol (DMPG : température de fluidité 23°C), et le phospholipide (II) est choisi dans le groupe constitué par la dimyristoylphosphatidylcholine (DMPC : température de fluidité 24°C), la dipalmitoylphosphatidylcholine (DPPC : température de fluidité 42°C), et la distéaroylphosphatidylcholine (DSPC : température de fluidité 58°C).

Selon un autre mode de mise en oeuvre préféré de la présente Invention, le rapport molaire entre le phospholipide (I) et le phospholipide (II) est compris entre 5:95 et 15:85.

Avantageusement, les liposomes convenant à la mise en oeuvre de l'Invention sont des liposomes multilamellaires, et ont un diamètre moyen compris entre 400 et 800 nm, de préférence environ 600nm. En vue de l'utilisation comme immunoadjuvant et/ou vecteur d'antigène, les Inventeurs ont constaté :
- qu'une certaine hétérogénéité du point de vue dimensionnel améliorait encore les propriétés immunoadjuvantes des préparations de liposomes conformes à l'Invention ;
- que, en particulier dans le cas de compositions destinées à l'administration orale, il était souhaitable que la préparation comprenne une proportion relativement importante de liposomes de grande taille (à savoir de diamètre supérieur à 500 nm).

Les liposomes convenant à la mise en oeuvre de l'Invention peuvent être préparés à partir d'un mélange de phospholipides tel que défini ci-dessus, par différentes méthodes connues en elles-mêmes [voir par exemple : Liposomes : A practical approach ; Practical approach series ; D. RICKWOOD et B.D. HAMES Eds, IRL press (1990)].

De préférence, on utilisera des phospholipides de synthèse, sans contamination entodoxinique (LPS<10 pg/µM de phospholipides). Les liposomes conformes à l'Invention ne contiennent pas de cholestérol, et ne présentent donc pas les inconvénients liés à son utilisation (pureté difficile à contrôler, potentiel toxique, activité immunosupressive).

Les liposomes convenant à la mise en oeuvre de l'Invention sont utilisables pour l'obtention de compositions immunomodulatrices, et en particulier immunostimulantes, ou de compositions immunogènes, et en particulier de vaccins, destinées à être administrées par voie muqueuse. Ces compositions induisent par exemple une réponse anticorps sérique et et/ou de type sécrétoire IgA et sont susceptibles d'induire une immunité cellulaire.

La présente Invention a pour objet des compositions immunomodulatrices, et en particulier immunoadjuvantes, caractérisées en ce qu'elles comprennent des liposomes tels que définis ci-dessus.

La présente Invention a également pour objet des compositions immunogènes, caractérisées en ce qu'elles comprennent des liposomes tels que définis ci-dessus, chargés avec au moins un antigène.

Par "liposomes chargés avec un antigène" on entend aussi bien des compositions dans lesquels l'antigène est solubilisé dans la phase aqueuse à l'intérieur du liposome, que des compositions dans lesquelles l'antigène est adsorbé sur la bicouche lipidique, à l'extérieur de celle-ci, ou des compositions dans lesquelles l'antigène (qui comprend dans ce cas une région hydrophobe) interagit avec la bicouche lipidique. L'antigène peut également être couplé par liaison covalente ou par liaison d'affinité (du type avidinebiotine), selon des techniques connues de l'homme de l'art, à des lipides, phospholipides, ou lipopeptides capables de s'insérer dans la bicouche lipidique.

Les propriétés adjuvantes particulières des liposomes conformes à l'Invention sont liées à la composition spécifique de leur bicouche lipidique. Ces liposomes peuvent donc être utilisés en association avec tout antigène incorporable dans des liposomes, en particulier des antigènes peptidiques, protéiques, glycoprotéiques, mais également des antigènes saccharidiques ou des acides nucléiques, etc....Ces antigènes peuvent être en particulier d'origine virale, parasitaire ou bactérienne ; par exemple, il peut s'agir d'antigènes de HIV tels que ceux décrits dans les Brevets et Demandes de Brevets publiées suivants : EP 138 667 ; FR 2 571 968 ; EP 239 425 ; WO 86/02383 ; d'antigènes du virus de l'hépatite B, tels que ceux décrits dans les Brevets et Demandes de Brevets publiées suivants : FR 2 464 269 ; EP 156 712, d'antigènes de Schistosomes, tels que par exemple ceux décrits dans les Brevets et Demandes de Brevets publiées suivants : FR 2 689 906 ; de Toxoplasmes, tels que par exemple ceux décrits dans les Brevets et Demandes de Brevets publiées suivants : FR 2 692 282 ; d'antigènes de *Bordetella pertussis* et *Bordetella anthracis* tels que ceux décrits dans les Brevets et Demandes de Brevets publiées suivants : WO 87/03301 ; FR 2 638 090, etc...

Il est possible de préparer d'abord des liposomes non chargés, puis de les charger avec l'antigène désiré, ou bien de procéder, en une seule étape, à la formulation des liposomes en présence de l'antigène et du mélange de phospholipides. La méthode la plus appropriée sera dans ce cas choisie en fonction de la nature de l'antigène, afin de respecter son intégrité structurelle.

Les liposomes pour la mise en oeuvre de l'Invention peuvent être préparés séparément des antigènes et subir un contrôle de fabrication indépendamment de l'antigène.

La composition immunogène finale sera obtenue par simple mélange du/ou des antigènes avec la préparation liposomale dans un véhicule pharmaceutiquement acceptable.

Les liposomes pour la mise en oeuvre de l'Invention peuvent être chargés avec plusieurs antigènes. Il est également possible de charger en outre les liposomes avec différentes substances susceptibles d'améliorer le pouvoir vaccinal de la préparation.

Par exemple, pour améliorer le pouvoir vaccinal par voie orale, on peut utiliser la sous-unité béta de la toxine cholérique, dont la capacité à se fixer préférentiellement au niveau des cellules M des plaques de Peyer permet d'induire une réponse IgA plus importante.

Pour améliorer le pouvoir vaccinal par voie nasale, on peut utiliser des adhésines, par exemple la protéine FHA (hémagglutinine filamenteuse) de *Bordetella pertussi*s, qui permet une meilleure intégration des liposomes dans les tissus et amplifie la réponse sécrétoire IgA.

Les liposomes selon l'Invention peuvent également être associés à des substances à activité immunomodulatrice, par exemple l'interféron gamma, les interleukines IL-1 et IL-2, le CGSF, le TNF (tumor necrosis factor).

Selon un mode de réalisation préféré des compositions immunogènes conformes à l'Invention, lesdites compositions sont administrables par voie orale.

Selon un autre mode de réalisation préféré des compositions immunogènes conformes à l'Invention, lesdites compositions sont administrables par voie intranasale.

Il est également possible, par exemple dans le cas de l'administration intranasale, de ne pas charger en antigène les liposomes, qui jouent alors uniquement un rôle d'adjuvant ; dans ce cas on peut administrer les liposomes et l'antigène soit simultanément soit séparément, l'administration des liposomes étant par exemple suivie dans un intervalle de 24 heures par une administration de l'antigène.

Les liposomes conformes à l'Invention ont été utilisés en association avec l'antigène Sm28GST (glutathion S-transférase de 28 kDa) de *Schistosoma mansoni*, afin d'obtenir un vaccin administrable par voie muqueuse et induisant une protection vis-à-vis de la parasitose à schistosomes.

L'antigène Sm28GST induit une immunité protectrice chez les mammifères, en réduisant le nombre de vers adultes et en diminuant la fécondité de ces vers. La réduction de la ponte induite par l'immunisation implique une réduction de la pathologie hépatique.

De récents travaux [GRZYCH et al., J. Immunol. 150, pp. 527-535, (1993)] ont montré l'implication probable d'anticorps de type IgA dans ce processus de protection. Ces IgA sont détectables dans le sérum et représentent surtout l'isotype majeur de la réponse immune sécrétoire. Toutefois l'administration de cet antigène par voie orale n'avait jusqu'à présent pas permis d'obtenir une réponse immune.

Lorsque cet antigène est administré par voie muqueuse avec des liposomes conformes à l'invention, on observe une réponse immmune importante, à la fois sérique et sécrétoire.

Des peptides immunodominants (peptides N-terminal, 10-43, 115-131, 190-211) de la Sm28GST peuvent également être utilisés pour charger les liposomes conformes à l'Invention. La séquence peptidique de l'antigène Sm28GST, ainsi que son obtention par génie génétique sont décrits dans la Demande Européenne 268 501 au nom de TRANSGENE S.A.

La présente Invention sera mieux comprise à l'aide du complément de description qui va suivre, qui se réfère à des exemples de préparation de liposomes, et de leur utilisation vaccinale, conformément à l'Invention.

Il doit être bien entendu toutefois que ces exemples sont donnés uniquement à titre d'illustration de l'objet de l'Invention dont ils ne constituent en aucune manière une limitation.

### EXEMPLE 1 - OBTENTION DES LIPOSOMES

### COMPOSITION DES LIPOSOMES UTILISES :

Trois sortes de liposomes sont réalisées. Ces liposomes présentent une rigidité variable suivant leur composition permettant ainsi une présentation différente de l'antigène au système immunitaire muqueux.
**DMPC/DMPG**
   dimiristoylphosphatidylcholine/dimiristoylphosphatidylglycérol
   Rapport : 9:1. Température de fusion : 24°C
**DPPC/DMPG**
   dipalmitoylphosphatidylcholine/dimiristoylphosphatidylglycérol
   Rapport : 9:1. Température de fusion : 37°C
**DSPC/DMPG**
   distéraoylphosphatidylcholine/dimiristoylphosphatidylglycérol
   Rapport : 9:1. Température de fusion : 56°C

Les poids moléculaires des phospholipides utilisés sont :
DMPC : 677,95
DPPC : 734,05
DSPC : 790,15
DMPG : 688,85.

Toute verrerie amenée à entrer en contact avec les phospholipides utilisés est préalablement lavée, rincée à l'eau déminéralisée et stérilisée par passage au four à 200°C durant 6 heures. Toutes les manipulations des poudres, la mise en solution et la manipulation de la verrerie ainsi que des transferts des solutions d'un récipient à l'autre sont réalisés en conditions stériles.

Après pesée, les différents phospholipides sont mis en solution à une concentration de 20 micromoles par millilitre dans l'alcool butylique tertiaire préalablement déshydraté sur tamis moléculaire et chauffé à 60°C pour le fluidifier. Les différentes solutions sont conservées dans des fioles de 30 ml teintées, à une température de -20°C.

Le mélange des phospholipides, c'est-à-dire, DMPC+ DMPG, DPPC+DMPG et DSPC+DMPG, est réalisé dans des fioles pour lyophilisation de 10 ml de contenance. Les solutions sont chauffées au bain-marie à 60°C pour les rendre parfaitement fluides. Un volume de 900 microlitres de dérivé de la phosphatidylcholine (DMPC, DPPC ou DSPC) sont déposés dans une fiole chaude (proche de 60°C), ainsi que 100 microlitres de la solution de DMPG. Mélangée à la pipette, la mixture est alors solidifiée régulièrement sur les parois de la fiole par rotation inclinée dans de la glace. Le rapport molaire du mélange obtenu est de 9 parts de dérivé de la phosphatidylcholine (DMPC, DPPC, ou DSPC) et de 1 part de DMPG.

Les fioles ouvertes sont alors mises dans un container stérile et le tout est conservé à -20°C en attente de la lyophilisation. La lyophilisation est réalisée durant 6 heures. Après cassage lent du vide dans des conditions stériles, les fioles sont rapidement bouchées et scellées. Les phospholipides ainsi préparés, apparaissent sous une forme de dépôt régulier sur les parois de la fiole. La formation de liposomes sera ultérieurement réalisée par hydratation du mélange, à l'aide de 1 ml de la solution contenant l'antigène à utiliser.

### EXEMPLE 2 -INCORPORATION ET RETENTION DE LA Sm28GST DANS LES LIPOSOMES

### Procédure utilisée

Les expérimentations ont été effectuées en utilisant en parallèle une solution de Sm28GST non marquée et une solution de Sm28GST marquée à l'iode radioactive (¹²⁵I) afin de permettre la mesure de son incorporation. La Sm28GST recombinante (fournie par la Société TRANSGENE) est incorporée dans les liposomes par hydratation des préparations sèches de phospholipides obtenues à l'exemple 1 avec une solution contenant la Sm28GST. Le mélange est incubé durant 45 secondes à une température de 60°C. Ce mélange est alors agité ou passé dans un bain à ultrasons pendant une minute puis incubé à température ambiante durant 15 minutes. Les liposomes ainsi formés et contenant la Sm28GST sont des vésicules unicellulaires de diamètre moyen d'environ 600 nm. Ces liposomes sont alors centrifugés à grande vitesse (13000 rpm, 20 mn) et lavés à l'eau distillée. Cette dernière opération est répétée trois fois pour éliminer toute trace de la molécule radiomarquée non incorporée dans les liposomes. Chaque culot de liposomes est finalement mis en suspension dans une solution saturée de bicarbonate de soude. La quantité de Sm28GST incorporée est alors déterminée par mesure de la radioactivité ¹²⁵I.

Les préparations de liposomes contenant la Sm28GST sont incubées à 4°C ou à 37°C durant 7 jours. La stabilité des différentes préparations est analysée après 1, 2, 3 et 7 jours, par mesure de la radioactivité ¹²⁵I dans le surnageant.

Le chauffage à 60°C de la Sm28GST pendant un certain temps risquait d'induire une dégradation de la structure tridimensionnelle de la protéine. Cette dénaturation peut être décelée par la variation de la reconnaissance de la Sm28GST après chauffage par différents anticorps monoclonaux dirigés contre les épitopes majeurs de la protéine, ainsi que par des anticorps polyclonaux dirigés contre la molécule entière. Afin d'avaluer les risques de dégradation, la Sm28GST a été chauffée à 60°C durant 5, 30 ou 60 minutes, puis la conservation de sa structure a été vérifiée par des anticorps monoclonaux dirigés contre la partie N-terminale (anti 24-43) ou C-terminales (anti 190-211) de la molécule ainsi que par un sérum dirigé contre la protéine totale.

### Résultats

L'incorporation de la Sm28GST dans les différents liposomes a été étudiée à 20°C et à 60°C.

Le pourcentage d'incorporation de la Sm28GST dans les liposomes (rapport protéine incorporée/protéine libre) à 20°C est le suivant :

| DMPC/DMPG | DPPC/DMPG | DSPC/DMPG |
|---|---|---|
| 5% | 5% | 15% |

Le pourcentage d'incorporation de la Sm28GST dans les liposomes (rapport protéine incorporée/protéine libre) à 60°C est le suivant :

| DMPC/DMPG | DPPC/DMPG | DSPC/DMPG |
|---|---|---|
| 35% | 40% | 50% |

L'incorporation de la Sm28GST dans les liposomes constitués de DMPC/DMPG ou de DPPC/DMPG est typique d'une incorporation de la protéine dans la phase aqueuse de la structure des liposomes. Le taux d'incorporation dans les liposomes DSPC/DMPG indique une interaction de la Sm28GST avec la bicouche phospholipidique du liposome. Cette interaction est sans doute due aux deux régions hydrophobes (acides aminés 130-150 et 150-180) de la protéine. Dans le cas d'une solution de Sm28GST à 5 mg/ml, la quantité incorporée dans les liposomes DSPC/DPMG est de 25 microgrammes pour 2 micromoles de phospholipides.

La rétention de la Sm28GST dans les trois sortes de liposomes est excellente, puisque aucune libération de la protéine n'a pu être décelée dans le surnageant des préparations après 7 jours d'incubation à 4°C ou à 37°C. Après 40 jours la rétention est encore supérieure à 99%.

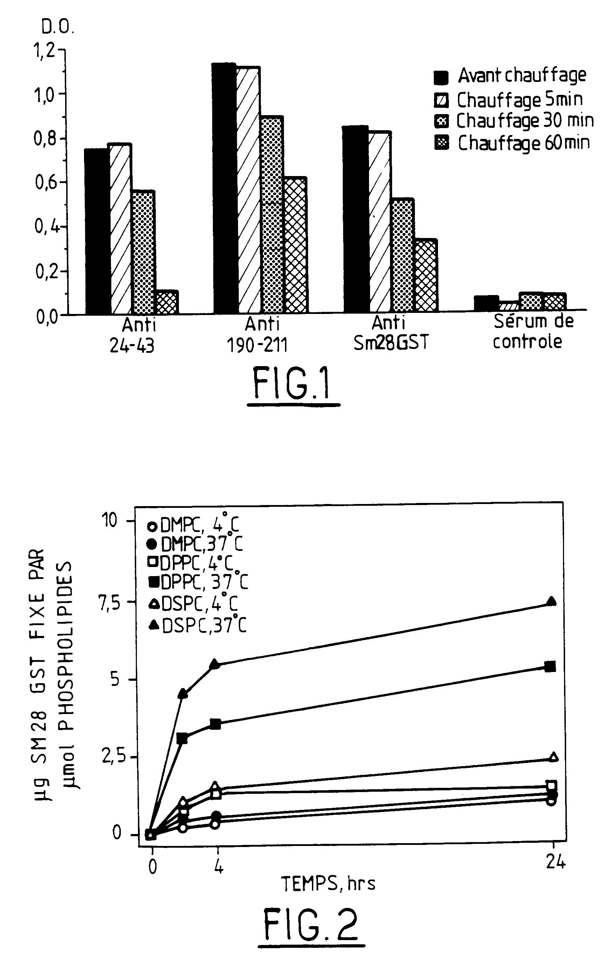

Comme le montre la figure 1, le chauffage à 60°C de la Sm28GST pendant 5 minutes ne provoque pas de changement dans la qualité antigénique de la molécule. Une dégradation de la structure peut être mise en évidence à partir de 30 minutes de chauffage.

L'incorporation de la Sm28GST dans les liposomes ne nécessitant qu'un chauffage d'une durée inférieure à une minute, le mode de préparation proposé respecte donc l'intégrité de la molécule.

Ce type de construction vaccinale est particulièrement approprié à la vaccination par voie orale.

### EXEMPLE 3 - INTEGRATION DE LA Sm28GST DANS LA BICOUCHE LIPIDIQUE DES LIPOSOMES

### Procédure utilisée

La Sm28GST est incubée avec les liposomes DMPC/DMPG, DPPC/DMPG ou DSPC/DMPG pendant 24 heures à 4 ou 37 °C. Les liposomes sont ensuite lavés à l'eau distillée trois fois, comme indiqué à l'exemple 2, pour éliminer toute trace de Sm28GST libre. Le contrôle de l'intégration est effectué en utilisant de la Sm28GST marquée à l'iode radioactive. La quantité de protéine fixée aux liposomes est alors évaluée par mesure de la radioactivité après 4 et 24 heures d'incubation. Les trois différentes préparations de liposomes ayant fixé la protéine sont incubées à 4 ou 37°C durant 7 jours. La stabilité de la fixation de la Sm28GST sur les différentes préparations liposomales est analysée après 1, 2, 3 et 7 jours, comme décrit à l'exemple 2.

### Résultats

La fixation de la Sm28GST est dépendante de la longueur de la chaîne de la phosphatidylcholine. La fixation sur les liposomes DMPC/DMPG et DPPC/DMPG est faible alors que celle observée avec les liposomes DSPC/DMPG est importante (voir Figure 2). Cette fixation sur les liposomes DSPC/DMPG est presque maximale après 4 heures d'incubation. La fixation de la Sm28GST est meilleure lorsque la réaction a lieu à une température de 37°C.

La stabilité de ces préparations est remarquable puisque le détachement de la Sm28GST de la surface des différentes préparations liposomales est négligeable après 7 jours d'incubation à 4 ou 37°C.

Ce type de construction vaccinale est approprié à la vaccination par voie nasale.

### EXEMPLE 4 - FIXATION DE LA Sm28GST BIOTYNYLEE AVEC LES LIPOSOMES CONTENANT DU PHOSPHATIDYL-ETHANOLAMINE-BIOTINE-AVIDINE

### Procédure utilisée

La Sm28GST marquée à l'iode radioactive (¹²⁵I) et couplée à la biotine (rapport 1/1) est incubée durant 24 heures à 37°C avec les différentes préparations de liposomes (DMPC/DMPG, DPPC/DMPG, DSPC/DMPG) en présence de 0, 5 ou 10 microgrammes de phosphatidyléthanolamine couplée à la biotine (rapport 1/1), incubée au préalable avec de l'avidine. Cette méthode provoque la formation d'un complexe "Biotine-Avidine-Biotine" permettant ainsi la fixation de la Sm28GST couplée à la surface des liposomes. Après trois lavages, la quantité de Sm28GST fixée est évaluée.

### Résultats

La fixation de la Sm28GST-biotine au "liposome-biotine-avidine" est identique quelque soit la composition en phospholipides du liposome, et est approximativement de 2 et 4 microgrammes par micromole de phospholipides contenant respectivement 5 et 10 microgrammes de phosphatidyléthanolamine.

Ce type de construction vaccinale est approprié à la vaccination par voie nasale.

### EXEMPLE 5 - ANTIGENICITE DE LA Sm28GST INCORPOREE DANS LES LIPOSOMES ET ADMINISTREE PAR VOIE ORALE.

### Procédure utilisée

Les liposomes sont préparées comme décrit à l'exemple 2.

Les animaux (souris OFl. C57bl/6 ou balb/c) reçoivent par gavage intestinal 0,25, 2,5 ou 25 microgrammes de Sm28GST incorporée dans les liposomes (préparations dosées à 0,25, 2,5 ou 25 microgrammes de Sm28GST pour deux micromoles de phospholipides) de différentes compositions, 3, 4, ou 5 fois à une semaine d'intervalle. Les anticorps de différents isotypes dirigés contre la Sm28GST sont recherchés dans le sérum et le lavage intestinal de ces souris, 1 semaine après la dernière administration. Un contrôle est effectué en utilisant des animaux auxquels a été administrée de l'eau distillée.

### Résultats

### a) Anticorps sériques

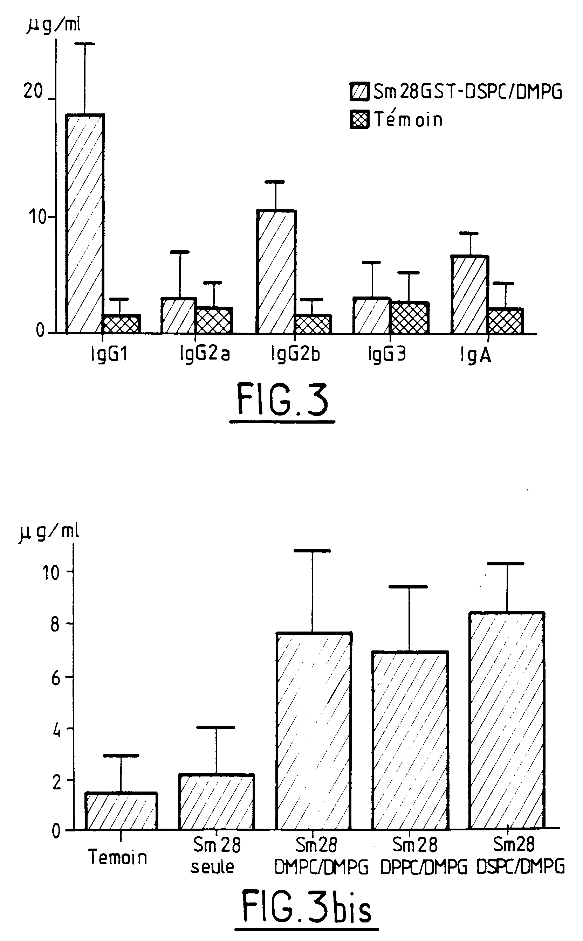

Une importante production d'anticorps anti Sm28GST est détectée dans le sérum après trois administrations par voie orale (Figure 3).

La figure 3 montre l'intensité et le profil isotypique de la réponse anticorps sérique des souris ayant reçu des liposomes DSPC/DMPG contenant 2,5 microgrammes de Sm28GST (une fois par semaine durant trois semaines). Ce profil indique que la formulation ainsi que la voie d'administration (orale) induit une réponse T de type Th2. Par ailleurs, une production d'anticorps de type IgA est observée.

Cette réponse anticorps est identique avec le DSPC/DMPG et le DPPC/DMPG du point de vue de son amplitude et de son profil isotypique. Une réponse sérique inférieure est obtenue avec le DMPC/DMPG mais avec un profil isotypique comparable.

### b) Anticorps sécrétoires

La détection d'anticorps de type IgA dans les sécrétions intestinales indique que l'administration de liposomes contenant la Sm28GST induit une bonne réponse sécrétoire. Alors que la Sm28GST libre donnée par voie orale n'induit aucune réponse sécrétoire, les trois formes de liposomes-Sm28GST provoquent des réponses sécrétoires comparables et importantes (figure 3bis).

La réponse anticorps, tous isotypes confondus, obtenue après quatre administrations est un peu plus importante que celle observée après trois administrations. Cinq administrations n'induisent pas une réponse statistiquement différente de celle obtenue avec quatre administrations.

### EXEMPLE 6 - PROTECTION CONTRE UNE INFECTION A SCHISTOSOMA MANSONI CHEZ LA SOURIS APRES VACCINATION PAR VOIE ORALE UTILISANT LA Sm28GST INCORPOREE DANS DES LIPOSOMES.

### Procédure utilisée

Utilisant le protocole d'administration indiqué à l'exemple 5, les souris (C57Bl/6, 6 semaines, femelles) ont reçu trois administrations par voie orale (2,5 ou 25 microgrammes de Sm28GST pour chaque administration) de Sm28GST contenue dans des liposomes DPPC/DMPG obtenus par le procédé décrit dans l'Exemple 2. Cinquante jours après la première administration, les souris sont infectées (voie transcutanée) par cent cercaires de *Schistosoma mansoni* provenant de la Guadeloupe. Cent jours plus tard, une perfusion totale sont pratiquée pour la récupération des vers adultes. Le foie ainsi que l'intestin grêle de chaque animal sont prélevés et digérés par une solution de potasse pour l'évaluation de la charge en oeufs.

### Résultats

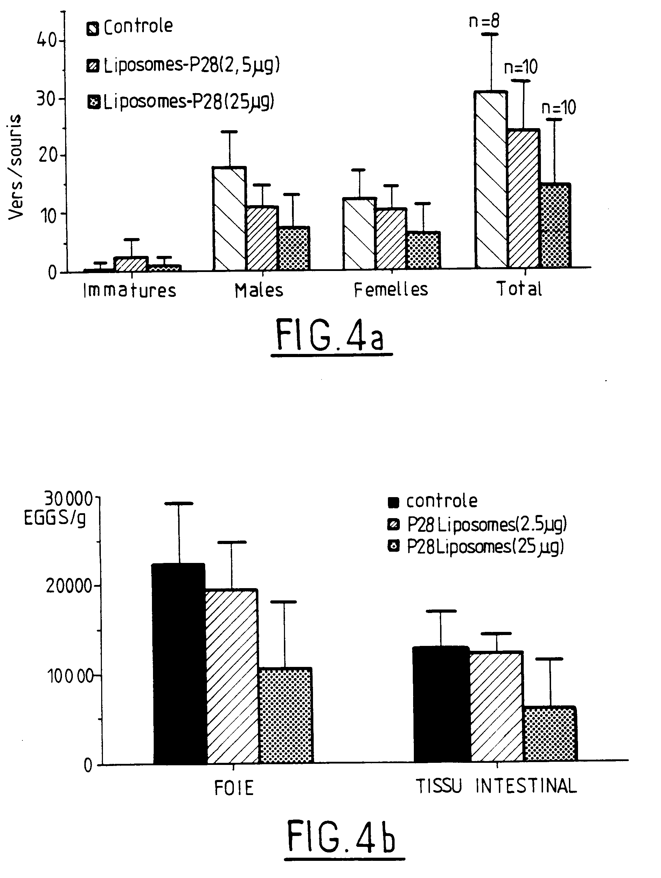

L'immunisation des souris avec 25 microgrammes de Sm28GST dans les liposomes induit une protection contre une infection parasitaire à *Schistosoma mansoni*, mise en évidence par la réduction de la charge en vers (Figure 4a) et par la réduction du nombre d'oeufs (Figure 4b).

Une réduction significative (p<0,01) de 52,3% du nombre de vers est obtenue dans le groupe de souris ayant reçu 25 µg de Sm28GST sous forme de liposomes, alors que les souris recevant 2,5 µg de Sm28GST sous forme de liposomes présentent une réduction de la charge vermineuse non significative de 22%. La charge en oeufs est également fortement réduite dans le groupe de souris ayant été immunisées avec 25 µg de Sm28GST contenus dans les liposomes. Cette diminution est de 53%, que ce soit dans le foie ou dans les tissus intestinaux.

### EXEMPLE 7 - VACCINATION NASALE

Des essais de vaccination nasale ont été réalisés avec des doses de 25 µg de Sm28GST, administrées sous forme de liposomes DSPC/DMPG obtenus par le procédé de l'Exemple 2. Des souris C57bl/6 ont reçu, par voie nasale, 3 fois à une semaine d'intervalle 25 µg de Sm28GST, libre ou intégrée dans les liposomes, en solution dans du tampon PBS. Des souris témoins ont reçu le même volume de PBS. L'instillation nasale est réalisée sous anesthésie générale et est d'un volume de 50 µl par administration.

Les résultats montrent que la Sm28GST libre est capable, lorsqu'elle est administrée par voie nasale, de provoquer une réponse sérique de l'ordre de 20 µg/ml d'anticorps de type IgG1. Par ailleurs, l'incorporation de la Sm28GST dans les liposomes DSPC/DMPG, le tout administré par voie nasale, provoque une réponse très puissante de profil Th2, ce qui correspond chez la souris à l'expression préférentielle, au niveau sérique, des isotypes IgG1 et IgG2b.

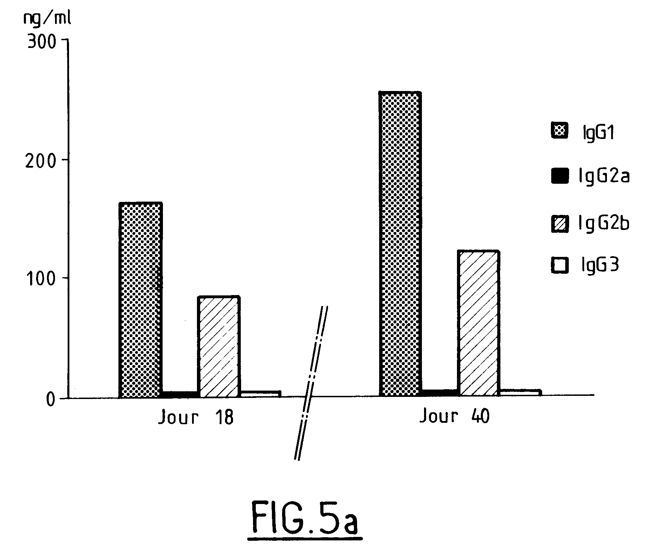

Ceci est illustré par la Figure 5a, qui représente la répartition des différents isotypes IgG1, IgG2a, IgG2b, et IgG3, dans la réponse immune spécifique sérique.

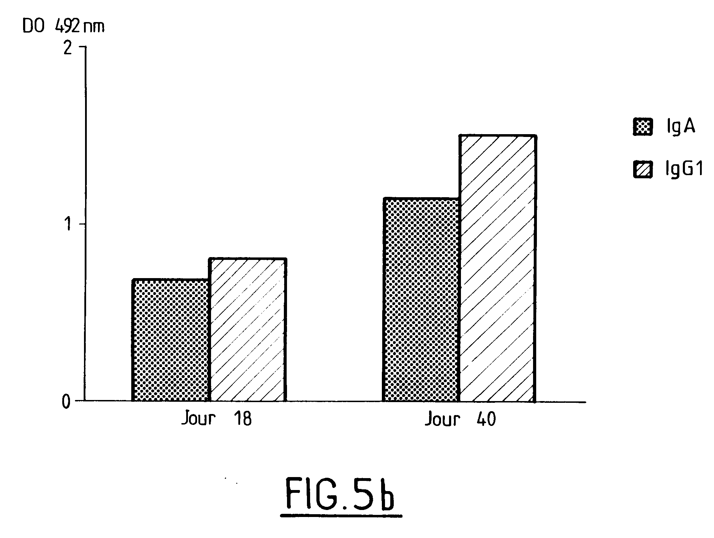

Cette réponse sérique est accompagnée d'une réponse importante de type IgA au niveau broncho-alvéolaire (les IgA spécifiques équivalent au minimum au 1/10 des IgA totaux broncho-alvéolaires). On observe également, à ce niveau, une forte proportion d'IgG1 spécifiques. Ceci est illustré par la Figure 5b, qui représente les IgA et IgG1 spécifiques détectées dans les lavages broncho-alvéolaires.

### EXEMPLE 8 - VACCINATION PAR VOIE SYSTEMIQUE

Des essais de vaccination par voies systémiques (voie intraveineuse, voie intramusculaire, voie sous-cutanée) ont été réalisées avec des doses de 25 µg de Sm28GST, administrées sous forme de liposomes DPPC/DMPG ou DSPC/DMPG obtenus par le procédé de l'exemple 2.

Des souris C57bl/6 ont reçu 3 fois à une semaine d'intervalle 25 µg de Sm28GST, libre, ou intégrée dans les liposomes, en solution dans du tampon PBS.

L'administration a été effectuée par injection, par voie intraveineuse, par voie intramusculaire, ou par voie sous-cutanée. L'injection a été réalisée sans anesthésie, et le volume injecté est de 0,2 ml, quelle que soit la voie d'injection.

Les résultats obtenus montrent que, par les trois voies systémiques d'injection, les liposomes chargés de Sm28GST induisent une réponse immune à la fois sérique et sécrétoire, alors que dans les mêmes conditions la Sm28GST libre en PBS n'induit aucune production spécifique d'anticorps.

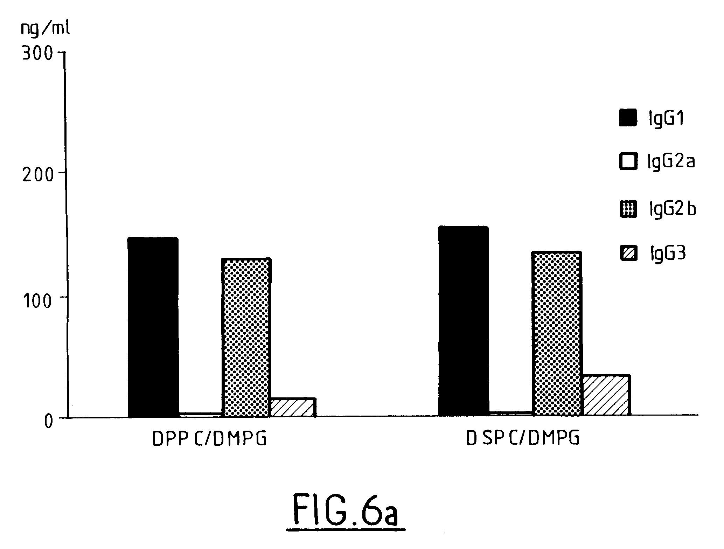

La voie intraveineuse semble la plus adéquate pour les deux formulations de liposomes utilisées ; en effet, l'injection intraveineuse de Sm28GST sous forme de liposomes provoque une réponse sérique importante et durable (présente au-delà de 70 jours) de profil Th2 et cela pour les deux formulations liposomales testées, à savoir DPPC/DMPG et DSPC/DMPG. Ceci est illustré par la Figure 6a, qui représente la répartition des différents isotypes IgG1, IgG2a, IgG2b, et IgG3, dans la réponse immune spécifique sérique, après injection intraveineuse des deux formulations de liposomes.

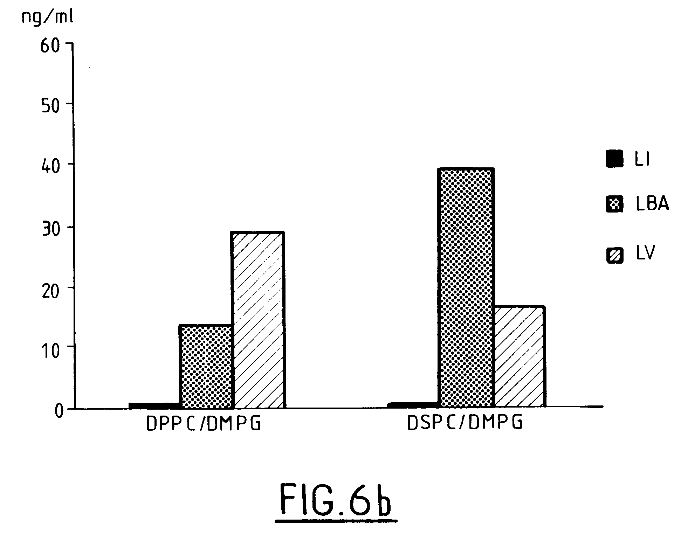

Cette réponse sérique est en outre accompagnée d'une réponse sécrétoire importante, mise en évidence dans les sécrétions broncho-alvéolaires et les sécrétions vaginales, mais pas dans les sécrétions intestinales (Figure 6b). Ceci est illustré par la Figure 6b, qui représente la quantité d'IgA spécifiques dans les lavages intestinaux (LI) bronchoalvéolaires (LBA) et vaginaux (LV), après injection intraveineuse des deux formulations de liposomes.

D'autre part, le type principal de réponse sécrétoire, à savoir broncho-alvéolaire ou vaginale, varie suivant le type de liposomes utilisé. La Sm28GST sous forme de liposomes DPPC/DMPG favorise une sécrétion vaginale d'IgA spécifiques, alors que la Sm28GST sous forme de liposomes DSPC/DMPG favorise la sécrétion d'IgA spécifiques au niveau broncho-alvéolaire.

## Revendications

1. Utilisation d'une préparation de liposomes constitués d'une ou plusieurs bicouche(s) lipidique(s) constituée(s) par un mélange d'un phospholipide (I) dont la température de fluidité est comprise entre 20 et 25°C, et d'un phospholipide (II) dont la température de fluidité est comprise entre 20° et 60°C, le rapport molaire entre le phospholipide (I) et le phospholipide (II) étant compris entre 5:95 et 30:70, en tant que principe actif immunoadjuvant pour l'obtention de vaccins.

2. Utilisation selon la revendication 1, caractérisée en ce que le phospholipide (I) est le dimyristoylphosphatidylglycérol, et le phospholipide (II) est choisi dans le groupe constitué par la dimyristoylphosphatidylcholine, la dipalmitoylphosphatidylcholine, et la distéaroylphosphatidylcholine.

3. Utilisation selon l'une quelconque des revendications 1 ou 2, caractérisée en ce que le rapport molaire entre le phospholipide (I) et le phospholipide (II) est compris entre 5:95 et 15:85.

4. Utilisation selon l'une quelconque des revendications 1 à 3, caractérisée en ce que la préparation de liposomes est utilisée pour l'obtention d'un vaccin administrable par voie orale.

5. Utilisation selon l'une quelconque des revendications 1 à 3, caractérisée en ce que la préparation de liposomes est utilisée pour l'obtention d'un vaccin administrable par voie intranasale.

6. Utilisation selon l'une quelconque des revendications 1 à 3, caractérisée en ce que la préparation de liposomes est utilisée pour l'obtention d'un vaccin administrable par voie d'injection intraveineuse, intramusculaire, ou sous-cutanée.

7. Utilisation selon l'une quelconque des revendications 1 à 6, caractérisée en ce que la préparation de liposomes est utilisée pour l'obtention d'un vaccin qui induit une réponse immunitaire de type IgA sécrétoire.

8. Utilisation selon l'une quelconque des revendications 1 à 6, caractérisée en ce que la préparation de liposomes est utilisée pour l'obtention d'un vaccin qui induit une réponse immunitaire de type cellulaire.

9. Composition immunomodulatrice, et en particulier immunoadjuvante, caractérisée en ce qu'elle est constituée par une préparation de liposomes telle que définie dans l'une quelconque des revendications 1 à 4.

10. Composition immunogène, caractérisée en ce qu'elle est constituée par une préparation de liposomes telle que définie dans l'une quelconque des revendications 1 à 4, lesquels liposomes sont associés avec au moins un antigène contre lequel on souhaite produire des anticorps spécifiques.

11. Composition immunogène selon la revendication 10, caractérisée en ce que l'antigène contre lequel on souhaite produire des anticorps spécifiques est la protéine Sm28GST, ou un peptide immunodominant de ladite protéine.

## Claims

1. A use of a preparation of liposomes consisting of one or several lipid bilayer(s) consisting of a mixture of a phospholipid (I) whose flow temperature is between 20 and 25°C and a phospholipid (II) whose flow temperature is between 20° and 60°C, the mole ratio of the phospholipid (I) to the phospholipid (II) being between 5:95 and 30:70, as immunoadjuvant active principle for obtaining vaccines.

2. The use as claimed in claim 1, wherein the phospholipid (I) is dimyristoylphosphatidylglycerol, and the phospholipid (II) is chosen from the group consisting of dimyristoylphosphatidylcholine, dipalmitoylphosphatidylcholine and distearoylphosphatidylcholine.

3. The use as claimed in either of claims 1 and 2, wherein the mole ratio of the phospholipid (I) to the phospholipid (II) is between 5:95 and 15:85.

4. The use as claimed in any one of claims 1 to 3, wherein the liposome preparation is used for obtaining a vaccine which is administerable orally.

5. The use as claimed in any one of claims 1 to 3, wherein the liposome preparation is used for obtaining a vaccine which is administerable intranasally.

6. The use as claimed in any one of claims 1 to 3, wherein the liposome preparation is used for obtaining a vaccine which is administerable by intravenous, intramuscular or subcutaneous injection.

7. The use as claimed in any one of claims 1 to 6, wherein the liposome preparation is used for obtaining a vaccine which induces a secretory IgA type immune response.

8. The use as claimed in any one of claims 1 to 6, wherein the liposome preparation is used for obtaining a vaccine which induces a cellular type immune response.

9. An immunomodulatory composition, and especially an immunoadjuvant composition, which consists of a liposome preparation as defined in any one of claims 1 to 4.

10. An immunogenic composition, which consists of a liposome preparation as defined in any one of claims 1 to 4, which liposomes are combined with at least one antigen against which it is desired to produce specific antibodies.

11. The immunogenic composition as claimed in claim 10, wherein the antigen against which it is desired to produce specific antibodies is the Sm28GST protein, or an immunodominant peptide of said protein.

## Patentansprüche

1. Verwendung einer Liposomenpräparation, gebildet aus einer oder mehreren Lipiddoppelschicht(en), die aus einem Gemisch eines Phospholipids (I), dessen Fluiditätstemperatur zwischen 20 und 25°C liegt, und einem Phospholipid (II), dessen Fluiditätstemperatur zwischen 20 und 60°C liegt, wobei das molare Verhältnis zwischen dem Phospholipid (I) und dem Phospholipid (II) zwischen 5:95 und 30:70 liegt, gebildet ist bzw. sind, als aktives Immunadjuvansprinzip, um Impfstoffe zu erhalten.

2. Verwendung nach Anspruch 1, dadurch **gekennzeichnet,** daß das Phospholipid (I) Dimyristoylphosphatidylglycerin ist, und daß das Phospholipid (II) aus der Gruppe bestehend aus Dimyristoylphosphatidylcholin, Dipalmitoylphosphatidylcholin, Distearoylphosphatidylcholin ausgewählt ist.

3. Verwendung nach einem der Ansprüche 1 oder 2, dadurch **gekennzeichnet,** daß das molare Verhältnis zwischen dem Phospholipid (I) und dem Phospholipid (II) zwischen 5:95 und 15:85 liegt.

4. Verwendung nach einem der Ansprüche 1 bis 3, dadurch **gekennzeichnet,** daß die Liposomenpräparation verwendet wird, um einen Impfstoff zu erhalten, der oral verabreicht werden kann.

5. Verwendung nach einem der Ansprüche 1 bis 3, dadurch **gekennzeichnet,** daß die Liposomenpräparation verwendet wird, um einen Impfstoff zu erhalten, der intranasal verabreicht werden kann.

6. Verwendung nach einem der Ansprüche 1 bis 3, dadurch **gekennzeichnet,** daß die Liposomenpräparation verwendet wird, um einen Impfstoff zu erhalten, der durch intravenöse, intramuskuläre oder subkutane Injektion verabreicht werden kann.

7. Verwendung nach einem der Ansprüche 1 bis 6, dadurch **gekennzeichnet,** daß die Liposomenpräparation verwendet wird, um einen Impfstoff zu erhalten, der eine Immunantwort vom sekretorischen IgA-Typ induziert.

8. Verwendung nach einem der Ansprüche 1 bis 6, dadurch **gekennzeichnet,** daß die Liposomenpräparation verwendet wird, um einen Impfstoff zu erhalten, der eine Immunantwort vom cellulären Typ induziert.

9. Immunmodulatorische Zusammensetzung, insbesondere eine Immunadjuvanszusammensetzung, dadurch **gekennzeichnet,** daß sie aus einer wie in einem der Ansprüche 1 bis 4 definierten Liposomenzusammensetzung gebildet wird.

10. Immunogene Zusammensetzung, dadurch **gekennzeichnet,** daß sie aus einer wie in einem der Ansprüche 1 bis 4 definierten Liposomenpräparation gebildet wird, wobei die Liposomen mit mindestens einem Antigen, gegen welches spezifische Antikörper gebildet werden sollen, assoziiert sind.

11. Immunogene Zusammensetzung nach Anspruch 10, dadurch **gekennzeichnet,** daß das Antigen, gegen welches spezifische Antikörper gebildet werden sollen, das Protein Sm28GST oder ein immundominantes Peptid dieses Proteins ist.
